# EUROPÄISCHE PATENTSCHRIFT

(11) **EP 1 728 469 B1**
(45) Veröffentlichungstag und Bekanntmachung des Hinweises auf die Patenterteilung: **16.12.2009**
(21) Anmeldenummer: 06009394.5
(22) Anmeldetag: 06.05.2006
(51) Int. Cl.: A61B 5/00, G06F 19/00, G01N 33/48

(54) **Bewertung der Glucosekonzentration zur Einstellung der Insulindosierung**
Evaluation of glucose concentration to adjust insulin dosage
Evaluation de la concentration de glucose pour régler le dosage insulinique

(30) Priorität: 16.07.2005 DE 102005033357; 04.06.2005 EP 05012091
(43) Veröffentlichungstag der Anmeldung: 06.12.2006
(73) Patentinhaber: Roche Diagnostics GmbH, 68305 Mannheim (DE); F.HOFFMANN-LA ROCHE AG, 4070 Basel (CH)
(72) Erfinder: Staib, Arnulf, Dr., 64646 Heppenheim (DE); Klötzer, Hans-Martin, Dr., 68163 Mannheim (DE)
(74) Vertreter: Mommer, Niels

(56) Entgegenhaltungen:
- EP-A- 1 281 351
- US-A- 4 731 726

## Beschreibung

Die Erfindung betrifft ein Verfahren zur Bewertung einer Serie von Werten der Glucosekonzentration einer Körperflüssigkeit eines Diabetikers für eine Einstellung der Dosierung von Insulingaben sowie eine zur Durchführung dieses Verfahrens geeignete Vorrichtung. Ein Verfahren mit den im Oberbegriff des Anspruchs 1 sowie eine Vorrichtung mit den im Oberbegriff des Anspruchs 16 angegebenen Merkmalen ist aus der US 4731726 bekannt.

Schwerwiegende langfristige Folgen des Diabetes mellitus (beispielsweise Erblinden aufgrund einer Retinopathie) können nur vermieden werden, wenn der Blutzuckerspiegel durch exakt dosierte Insulingaben ständig innerhalb enger Grenzen gehalten wird, die denen einer gesunden Person entsprechen. Insulinpflichtige Diabetiker müssen deshalb mehrmals täglich ihre Blutglucosekonzentration messen und sich Insulingaben in der jeweils benötigten Menge verabreichen.

Die optimale Dosierung von Insulingaben hinsichtlich Menge und Häufigkeit läßt sich nicht ohne weiteres aus Meßwerten der Blutglucosekonzentration ableiten. In der Praxis beruhen die gewählten Insulindosierungen zu einem erheblichen Teil auf Erfahrungswerten des behandelnden Arztes oder auch des Patienten selbst. Typischerweise wird für einen Diabetiker von einem Arzt ein Dosierungsplan erstellt, der einerseits Menge und Häufigkeit von Insulingaben zur Deckung eines Insulingrundbedarfs vorgibt und ferner Instruktionen enthält, wie als Reaktion auf erhöhte Meßwerte der Blutglucosekonzentration und Mahlzeiten zusätzliche Insulingaben dosiert werden sollen. Insulingaben zur Deckung des Insulingrundbedarfs werden in diesem Zusammenhang als Basalrate und zusätzliche Insulingaben im Zusammenhang mit Mahlzeiten als Bolus bezeichnet.

Die allgemeine Dosierungsanweisung, nach der ein Diabetiker unter Berücksichtigung von Meßwerten der Blutglucosekonzentration die Dosierung der zu verabreichenden Insulingaben ermittelt, bezeichnet man als Einstellung. Es besteht ein erheblicher Bedarf eine vorgenommene Einstellung zu bewerten, um diese gegebenenfalls korrigieren oder an eine Änderung der Lebensgewohnheiten oder des allgemeinen Gesundheitszustandes eines Patienten anpassen zu können.

Eine suboptimale Einstellung der Dosierung von Insulingaben läßt sich selbst anhand einer Serie von Meßwerten der Blutglucosekonzentration nicht ohne weiteres erkennen, da auch bei gesunden Menschen die Blutglucosekonzentration im Tagesverlauf starken Schwankungen unterliegt. In dem Artikel von N. Weintrob et al. "Glycemic patterns detected by continuous subcutaneous glucose sensing in children and adolescents with Type 1 Diabetes Mellitus treated by multiple injections vs continuous subcutaneous insulin infusion", Arch. Pediatr. Adolesc. 158, 677 (2004) wird der Ansatz verfolgt, kontinuierlich gemessene Blutglucosekonzentrationen hinsichtlich der Einstellung der Dosierung von Insulingaben anhand von Zeitintegralen der Blutglucosekonzentration zu bewerten.

Bei dieser Vorgehensweise wird für einen Zeitraum Δt von mehreren Tagen eine Fläche bestimmt, die oberhalb einer durch einen Schwellenwert von 180 mg/dl vorgegebenen Basislinie und unter der Kurve des zeitlichen Blutglucoseverlaufs liegt. Diese Fläche wird durch den Zeitraum Δt dividiert, um einen Parameter zu erhalten, der eine hyperglykämische Störung des Glukosestoffwechsels kennzeichnet. In entsprechender Weise wird ein Parameter bestimmt, der hypoklykämische Störungen des Glukosestoffwechsels kennzeichnet, indem eine Fläche zwischen einer durch einen unteren Schwellenwert von 70 mg/dl vorgegebenen Basislinie und der Kurve des zeitlichen Blutglukoseverlaufs für Zeiten bestimmt wird, zu denen die Blutglucosekonzentration diesen Schwellenwert unterschreitet.

Das bekannte Verfahren wird als Area-Under-Curve (AUC) Berechnung bezeichnet. Ein ähnliches Verfahren ist zur Diagnose von Diabetes auch aus der WO 2004/043230 A2 bekannt. Darin wird empfohlen ergänzend weitere Merkmale eines gemessenen Verlaufs der Blutglucosekonzentration, beispielsweise die Steigung, auszuwerten.

Mit derartigen Verfahren läßt sich zwar generell eine Aussage ermitteln ob ein insulinpflichtiger Diabetiker gut eingestellt ist. Wesentlich für eine gezielte Therapieempfehlung oder eine Optimierung der Einstellung ist aber die Erkennung von Perioden glykämischer Instabilitäten in dem Glucoseprofil und deren kausale Zuordnung zu einer erfolgten Insulindosierung oder Nahrungsaufnahme. Insbesondere für Personen mit einer schwankenden Insulinsensivität (sogenannte brittle Diabetes) ist es mit bekannten Verfahren praktisch nicht möglich eine optimale Einstellung der Insulindosierung zu erreichen.

Aufgabe der Erfindung ist es deshalb, einen Weg aufzuzeigen, wie eine Serie von Werten der Glucosekonzentration einer Körperflüssigkeit eines Diabetikers zur Einstellung der Dosierung von Insulingaben besser bewertet werden kann, so daß mit geringerem Aufwand eine optimale Insulindosierung für Diabetiker erreicht werden kann. Insbesondere soll auch bei Patienten mit schwankender Insulinsensivität durch eine verbesserte Bewertung eine Serie von Meßwerten der Glucosekonzentration die Einstellung der Dosierung von Insulingaben verbessert werden können.

Diese Aufgabe wird gelöst durch ein Verfahren mit den im Anspruch 1 angegebenen Merkmalen.

Die Aufgabe wird ferner gelöst durch eine Vorrichtung mit den im Anspruch 15 angegebenen Merkmalen.

Die Körperflüssigkeit, deren Glucosekonzentrationswerte im Rahmen der Erfindung betrachtet werden, kann beispielsweise interstitielle Flüssigkeit oder spektroskopisch vermessene Augenflüssigkeit sein. Ohne Beschränkung der Allgemeinheit wird im Folgenden von Blutglucosekonzentration gesprochen, da Blut wohl die Körperflüssigkeit ist, die am häufigsten zur Untersuchung des Glucosestoffwechsels herangezogen wird. Im Rahmen der Erfindung kann jedoch ebensogut die Glucosekonzentration einer beliebigen anderen Körperflüssigkeit herangezogen werden.

Durch die Verwendung einer Wertungsfunktion können Werte der Blutglucosekonzentration, die besonders stark von dem vorgegebenen Sollbereich, beispielsweise 70 mg/dl bis 180 mg/dl, abweichen bei der Berechnung des Störungsparameters mit einem größerem Gewicht berücksichtigt werden als Konzentrationswerte, die nur geringfügig von dem Sollbereich abweichen. Erfindungsgemäß kann deshalb auf der Grundlage von Funktionswerten der Wertungsfunktion ein Störungsparameter ermittelt werden, der eine Störung des gesuchten Glucosestoffwechsels zutreffender charakterisiert und folglich eine Verbesserung der Einstellung des Dosierung der Insulingaben ermöglicht.

In diesem Zusammenhang ist unter der Störung des untersuchten Glukosestoffwechsels selbstverständlich nicht die krankheitsbedingte Störung zu verstehen, die Insulingaben erforderlich macht, sondern die trotz der Insulingaben noch vorhandene, nicht kompensierte Störung. Der erfindungsgemäß ermittelte Störunsparameter kennzeichnet also eventuell vorhandene Unterschiede zwischen dem zeitlichen Verlauf der Blutglucosekonzentrationswerte eines durch Insulingaben behandelten Diabetikers und einer gesunden Vergleichsperson.

Wichtig ist, daß der erfindungsgemäß ermittelte Störungsparameter nicht notwendigerweiße eine eindimensionale Größe, d. h. eine natürliche oder reelle Zahl, sondern vorzugsweise ein Satz von Werten ist, die jeweils verschiedene Aspekte der Störung charakterisieren. Beispielsweise kann der Störungsparameter ein Wertepaar sein, bei dem ein erster Wert die Schwere von hyper- oder hypoglykämischen Abweichungen von dem Sollbereich und ein zweiter Wert, deren zeitliche Dauer charakterisiert. Dieses Wertepaar könnte beispielsweise um einen dritten Wert ergänzt werden, der die Häufigkeit derartiger Störungen angibt.

Besonders vorteilhaft läßt sich die Erfindung zur Bewertung von Konzentrationswerten nutzen, die über längere Zeiträume, beispielsweise mehrere Tage, in relativ kurzen Zeitabständen von wenigen Minuten gemessen wurden. Meßtechniken für entsprechende Messungen sind in der Literatur unter dem Stichwort continuous monitoring (CM) beschrieben. Steht eine ausreichend lange Serie von Konzentrationswerten, die in hinreichend kleinen Abständen gemessen wurden, zur Verfügung, so läßt sich das erfindungsgemäße Verfahren zunächst für einzelne Zeitintervalle, die in dem untersuchten Zeitraum enthalten sind, anwenden und aus den für die einzelnen Zeitintervalle ermittelten Resultaten in einem weiteren Schritt, vorzugsweise durch statistische Methoden, der Störungsparameter ermitteln.

Bei dieser Vorgehensweise wird aus Störungswerten des betreffenden Intervalls zunächst eine Störungskennzahl berechnet und in einem weiteren Schritt aus den Störungskennzahlen der einzelnen Intervalle der Störungsparameter ermittelt. Für Fälle, in denen nur eine relativ geringe Anzahl von Konzentrationswerten zur Verfügung steht und deshalb nur ein einziges Zeitintervall ausgewertet wird, kann die Störungskennzahl mit dem Störungsparameter übereinstimmen.

Im einfachsten Fall handelt es sich bei den erfindungsgemäß ausgewerteten Konzentrationswerten jeweils um Meßwerte. Diese können in geeigneter Weise, beispielsweise durch Anwendung statistischer Verfahren oder geeigneter Filteralgorithmen, aufbereitet werden, um Konzentrationswerte zu bestimmen.

Für die konkrete Form der erfindungsgemäß zu verwendeten Wertungsfunktion gibt es eine Vielzahl von Möglichkeien. Als Wertungsfunktionen können beispielsweise im Bereich von Optimierungsproblemen gebräuchliche Penalty-Funktionen verwendet werden.

Bevorzugt hat die Wertungsfunktion in einem Sollbereich der Werte der Blutglucosekonzentration eine geringere Steigung als in einem an den Sollbereich angrenzenden Bereich. Bevorzugt liegt dieser Sollbereich zwischen 50 mg/dl und 250 mg/dl, vorzugsweise zwischen 70 mg/dl und 180 mg/dl. Bei einer entsprechend gewählten Wertungsfunktion, insbesondere einer nichtlinearen Wertungsfunktion, kann der Sollbereich auch wesentlich kleiner, im Extremfall sogar als Sollwert von beispielsweise 120 mg/dl, gewählt werden.

Bevorzugt folgen die Zeitpunkte t₁ bis tₙ der Werte der Glukosekonzentration g(t₁) bis g(tₙ) in Zeitabständen von weniger als 20 min aufeinander, besonders bevorzugt in Zeitabständen von weniger als 10 min, insbesondere in Zeitabständen von weniger als 5 min.

Weitere Einzelheiten und Vorteile der Erfindung werden anhand eines Ausführungsbeispiels unter Bezugnahme auf die beigefügten Zeichnungen erläutert. Die darin offenbarten Merkmale können einzeln oder in Kombination verwendet werden, um bevorzugte Ausgestaltungen der Erfindung zu schaffen. Es zeigen:
- Fig. 1: ein Beispiel einer Wertungsfunktion;
- Fig. 2: weitere Beispiele einer Wertungsfunktion;
- Fig. 3: ein Beispiel einer Serie von Werten der Blutglucosekonzentration eines Diabetikers;
- Fig. 4: Mittelwerte und Standardabweichungen der Funktionswerte der Wertungsfunktion für überlappende Zeitintervalle von jeweils 12 Stunden für die in Fig. 3 dargestellte Serie;
- Fig. 5: Standardabweichungen und Mittelwerte von Funktionswerten der Wertungsfunktion für verschiedene Probanden; und
- Fig. 6: eine schematische Darstellung einer Vorrichtung zur Durchführung eines erfindungsgemäßen Verfahrens.

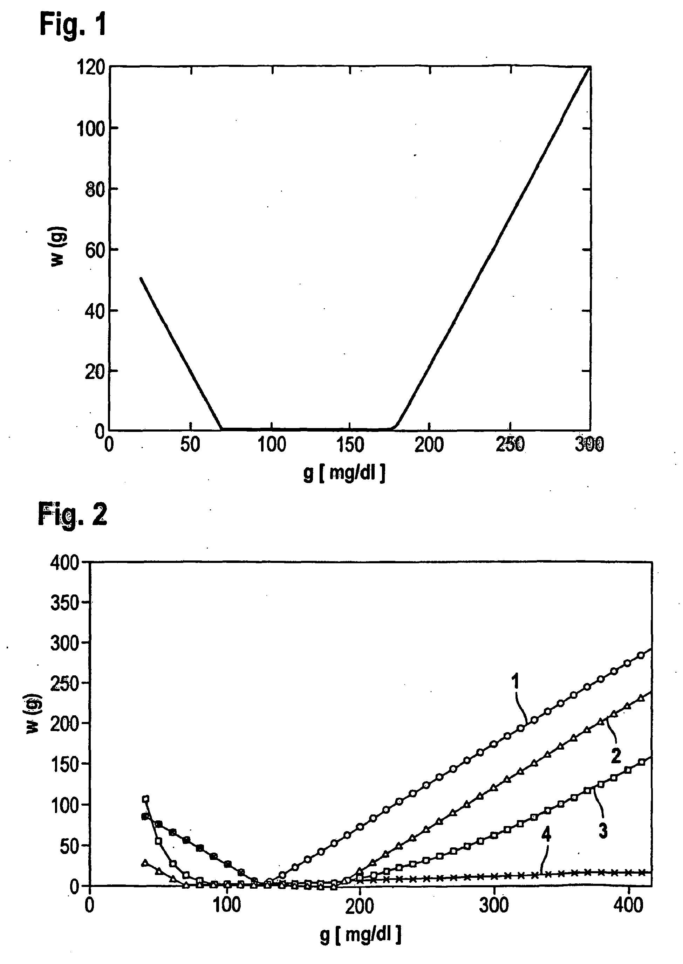

In Fig. 1 ist ein Beispiel einer Wertungsfunktion w dargestellt, mit der aus Werten der Blutglucosekonzentration g Störungswerte w(g) berechnet werden. Eine wesentliche Eigenschaft der Wertungsfunktion besteht darin, daß sie in einem Sollbereich der Blutglucosekonzentration eine geringere Steigung als außerhalb des Sollbereichs hat. Bei dem dargestellten Beispiel wurde als Sollbereich ein Normoglykämischer Bereich von 70 mg/dl bis 180 mg/dl gewählt.

Die dargestellte Wertungsfunktion steigt außerhalb des Sollbereichs linear an und hat in dem Sollbereich einen konstanten Verlauf. Es ist jedoch nicht erforderlich, daß als Wertungsfunktion eine solche abschnittsweise lineare Funktion gewählt wird. Möglich ist es auch, daß die Wertungsfunktion außerhalb des Sollbereichs nicht linear ansteigt. Auch ist nicht unbedingt erforderlich, daß die Wertungsfunktion in dem Sollbereich konstant ist. Beispielsweise kann als Wertungsfunktion auch eine parabelförmige Funktion gewählt werden, deren Scheitelpunkt sich bei etwa 120 mg/dl befindet.

Weitere Beispiele geeigneter Wertungsfunktionen sind in Fig. 2 dargestellt. Die Wertungsfunktion 1 ist ein Beispiel für eine Wertungsfunktion, bei welcher der Sollbereich stark verkleinert wurde, so daß er in dem dargestellten Extremfall zu einem Punkt zusammengeschrumpft ist. Die Funktionen 2 und 3 sind Beispiele für asymmetrische Wertungsfunktionen, die hypoglykämische Abweichungen von dem Sollbereich stärker gewichten als hyperglykämische Abweichungen. Die Wertungsfunktionen 2 und 3 sind zugleich Beispiele für nichtlineare Wertungsfunktionen. Bei der Wertungsfunktion 3 handelt es sich um den Schlichtkrull Index. Die Wertungsfunktion 4 stimmt in dem hypoglykämischen Bereich mit der Wertungsfunktion 1 überein. In dem hyperglykämischen Bereich steigt die Wertungsfunktion 4 zunächst in einem an den Sollbereich angrenzenden Abschnitt an, geht im weiteren jedoch in einen konstanten Verlauf über. Die Funktion 4 ist deshalb auch ein Beispiel dafür, daß eine Wertungsfunktion nicht überall außerhalb des Sollbereichs eine größere Steigung als innerhalb des Sollbereichs haben muß. Es genügt vielmehr, daß die Steigung in einem an den Sollbereich angrenzenden Bereich größer als in dem Sollbereich selbst ist.

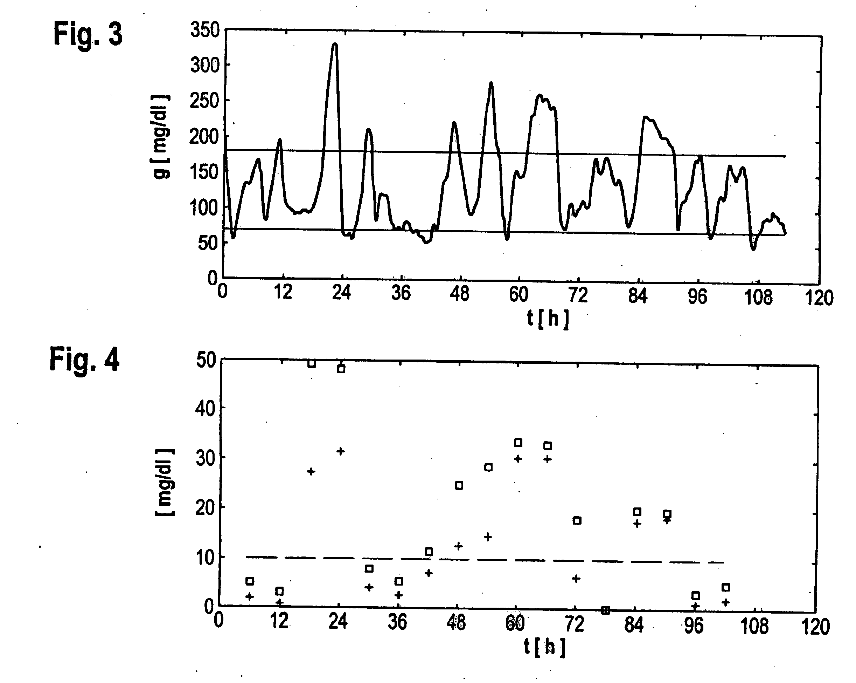

Fig. 3 zeigt ein Beispiel einer Serie von Werten der Blutglucosekonzentration eines Diabetikers über einen Zeitraum von etwa 110 Stunden. Die dargestellten Konzentrationswerte wurden in Abständen von etwa 5 Minuten ermittelt. Der Sollbereich der in Fig. 1 dargestellten Wertungsfunktion ist in Fig. 2 durch horizontal verlaufende Linien bei Blutglucosekonzentrationen von 70 mg/dl beziehungsweise 180 mg/dl angedeutet. Fig. 3 zeigt, daß die Blutglucosekonzentration im Tagesverlauf erheblichen Schwankungen unterliegt und bei einem Diabetiker sowohl hyper- als auch hypoglykämische Perioden auftreten, die durch eine optimale Einstellung der Dosierung von Insulingaben verhindert werden sollen.

Zur Auswertung wurden aus den Konzentrationswerten der in Fig. 3 dargestellten Serie zunächst Störungswerte als Funktionswerte der anhand von Fig. 1 beschriebene Wertungsfunktion berechnet. Diese Störungswerte wurden jeweils für 12 stündige Zeitintervalle ausgewertet, indem ein Mittelwert der Störungswerte des jeweiligen Zeitintervalls und die dazugehörende Standardabweichung berechnet wurden. Bevorzugt wird als Mittelwert das arithmetische Mittel gebildet.

In Fig. 4 sind die für zwölfstündige Zeitintervalle berechneten Mittelwerte der Störungswerte w(g) jeweils durch Kreuze (+) und die dazugehörenden Standardabweichungen durch kleine Kästchen (□) dargestellt. Bevorzugt werden die Zeitintervalle überlappend gewählt. Bei dem dargestellten Ausführungsbeispiel überlappen aufeinanderfolgende Zeitintervalle jeweils um 6 Stunden. Die Zeitintervalle können im Einzelfall an den Tagesablauf eines Patienten angepaßt werden, so daß sie beispielsweise mit dessen nächtlichen Schlafperioden oder Wachzeiten übereinstimmen. Günstig ist auch eine Wahl der Zeitintervalle gemäß prä- und postprandialen Phasen, so daß therapeutisch relevante Zusammenhänge leichter aufgezeigt werden können.

Die in Fig. 4 dargestellten Mittelwerte und die dazugehörenden Standardabweichungen stellen als Wertepaar eine Störungskennzahl für das jeweilige Zeitintervall dar, die Art und/oder Schwere einer Störung des untersuchten Glucosestoffwechsels charakterisiert. Dabei ist es selbstverständlich nicht zwingend, daß als Komponenten der Störungskennzahlen die Mittelwerte bzw. die Standardabweichungen selbst verwendet werden. Ebensogut läßt sich ein beliebiges Maß für die Mittelwerte bzw. die Standardabweichungen als Komponente der Störungskennzahlen verwenden. Im einfachsten Fall ist das Maß ein Vielfaches des Mittelwertes bzw. der Standardabweichung oder wird durch Addition eines konstanten Terms aus dem Mittelwert bzw. der Standardabweichung berechnet.

Aus den Störungskennzahlen der einzelnen Zeitintervalle wird durch eine statistische Auswertung ein Störungsparameter ermittelt, der einer von mindestens zwei vorgegebenen Klassen zugeordnet wird. Diesen Klassen sind jeweils Empfehlungen zur Einstellung der Dosierung der Insulingaben zugeordnet, so daß auf diese Weise die Einstellung der Dosierung der Insulingaben verbessert werden kann.

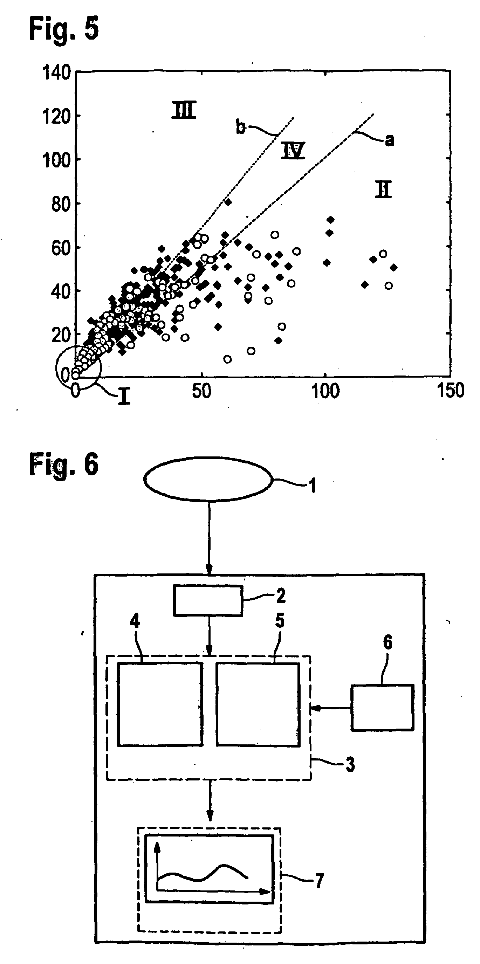

In Fig. 5 sind Standardabweichungen und Mittelwerte von Funktionswerten der Wertungsfunktion eingetragen, die auf die beschriebene Weise ermittelt wurden. Die Ordinate gibt die Standardabweichungen und die Abszisse die Mittelwerte der Funktionswerte der Wertungsfunktion für Zeitintervalle von jeweils 12 Stunden an. In Fig. 5 sind Ergebnisse der Auswertung von Serien von Blutglucosekonzentrationswerten dargestellt, die an 45 Probanden gemessen wurden. Jede Serie wurde dabei über einen Zeitraum von etwa 5 Tagen gemessen. Ergebnisse für Typ 1 Diabetiker sind dabei durch Rauten, Ergebnisse für insulinpflichtige Typ 2 Diabetiker durch Kreise dargestellt.

Fig. 5 zeigt, daß zwischen Ergebnissen für Typ 1 Diabetiker und Ergebnissen für Typ 2 Diabetiker kein signifikanter Unterschied besteht. Dies ist nicht überraschend, da bei der Einstellung der Dosierung von Insulingaben in beiden Fällen im wesentlichen die selben Schwierigkeiten auftreten. Wie anhand von Fig. 4 erläutert wurde, stellen ein Mittelwert und die dazugehörende Standardabweichung der Funktionswerte der Wertungsfunktion als Wertepaar eine Störungskennzahl für das jeweilige Zeitintervall einer untersuchten Serie von Konzentrationswerten dar. Diese Störungskennzahlen lassen sich in vier verschiedene Klassen einteilen.

Zu einer ersten Klasse I gehören Störungskennzahlen, die in Fig. 5 in einem Kreis um den Ursprung des Koordinatensystems eingezeichnet sind. Störungskennzahlen der Klasse I sind so gering, daß von einer optimalen Einstellung der Dosierung der Insulingaben ausgegangen werden kann. In diesen Fällen ist keine Änderung der Einstellung erforderlich.

Einer zweiten Klasse II werden Störungskennzahlen zugeordnet, die in Fig. 5 unterhalb einer gestrichelt dargestellten Ursprungsgraden a dargestellt sind. Zu der Klasse II gehören Fälle, in denen die Mittelwerte der Störungswerte größer sind, als die dazugehörenden Standardabweichungen. Störungskennzahlen der Klasse II deuten deshalb auf Fälle hin, in denen für längere Zeiträume eine deutliche Abweichung der Blutglucosekonzentrationswerte von dem Sollbereich aufgetreten ist. Störungskennzahlen der Klasse II lassen folglich auf eine schlecht eingestellte Basalrate der Insulingaben schließen. Handelt es sich bei der beobachteten Abweichung der Blutglucosekonzentration von dem Sollbereich um hypoglykämische Abweichungen, so ist die Basalrate zu verringern, handelt es sich um hyperglykämische Abweichungen, so ist die Basalrate zu erhöhen.

Einer dritten Klasse III werden Störungskennzahlen zugeordnet, die in Fig. 5 oberhalb einer punktiert eingezeichneten Ursprungsgraden b dargestellt sind. Störungskennzahlen der Klasse III bedeuten, daß die Standardabweichung der Funktionswerte der Wertungsfunktion, d. h. die Störungswerte, in dem untersuchten Zeitintervall von dem Mittelwert wesentlich größer ist als der Mittelwert selbst, beispielsweise 20% größer ist. Zur Klasse III gehören also Fälle, in denen die Störungswerte starken kurzfristigen Fluktuationen unterliegen. Dies bedeutet, daß die zugrunde liegende Serie von Konzentrationswerten durch kurzfristige Abweichungen von dem Sollbereich geprägt ist. In der Regel treten derartige Abweichungen im Zusammenhang mit Mahlzeiten oder körperlicher Anstrengung auf, so daß zur Optimierung der Einstellung der Boli, also die zusätzlichen Insulingaben im Zusammenhang mit Mahlzeiten oder körperlicher Betätigung, angepaßt werden sollte.

Einer vierten Klasse IV werden Störungskennzahlen zugeordnet, die in Fig. 5 zwischen der punktierten Ursprungsgeraden b und der gestrichelten Ursprungsgeraden a dargestellt sind. In derartigen Fällen sind die Mittelwerte und die Standardabweichungen der Funktionswerte der Wertungsfunktion in dem betrachteten Zeitintervall etwa gleich groß, so daß sowohl die Basalrate als auch der Bolus der Insulingaben zu überprüfen und anzupassen ist.

Bei dem anhand von Fig. 5 erläuterten Beispiel stellt der Index der jeweiligen Klasse (d. h. die Zahl I, II, III oder IV) den Störungsparameter dar, der durch eine statistische Auswertung der Störungswerte ermittelt wurde. Jedem dieser Störungsparameter, d. h. jeder dieser Klassen, ist jeweils eine qualitative Empfehlung zugeordnet, wie die Einstellung der Dosierung von Insulingaben anzupassen ist. Werden zusätzlich zu einer Serie von Konzentrationswerten weitere für den zeitlichen Verlauf der Blutglucosekonzentration relevante Daten, insbesondere über verabreichte Insulingaben und aufgenommene Broteinheiten, erfaßt und ausgewertet, können unter Verwendung dieser Daten und der den Klassen zugeordneten Empfehlungen auch die benötigten Insulindosen und die Zeitpunkte, zu denen sie verabreicht werden sollen, berechnet werden. Dazu sind die Abweichungen der Blutglucosekonzentrationswerte von dem Sollbereich quantitativ einzubeziehen. Alternativ können die Störungskennzahlen für die jeweilige Klasse zunächst qualitativ ausgewertet werden, wobei anschließend der Grad der Abweichung erfaßt und zur Berechnung der Insulindosen herangezogen werden kann.

Bei dem im Vorhergehenden beschriebenen Verfahren wurde der Störungsparameter auf der Grundlage der Mittelwerte der Störungswerte und der Standardabweichung der Störungswerte berechnet. Ergänzend oder alternativ kann der Störungsparameter oder einige seiner Komponenten beispielsweise auch dadurch ermittelt werden, daß die Störungswerte jeweils einem von mehreren Stoffwechsel-Zuständen zugeordnet werden und ermittelt wird, wie lange die verschiedenen Zustände jeweils andauern und/oder mit welcher Häufigkeit Zustandswechsel auftreten. Eine Komponente des Störungsparameters kann dann beispielsweise ein Maß für die Zeitspanne sein, über die der betreffende Zustand andauert. Eine weitere Komponente des Störungsparameters kann ein Maß für die Häufigkeit der Zustandswechsel sein.

Störungswerte können Zuständen des Glucosestoffwechsels beispielsweise anhand von vorgegebenen Schwellenwerten zugeordnet werden. Anstelle einer Störungsfunktion, wie sie in den Figuren 1 und 2 dargestellt ist, kann bei einer derartigen Vorgehensweise auch eine Stufenfunktion verwendet werden, die beispielsweise in einem Sollbereich den Wert 0 (normoglykämischer Bereich), unterhalb des Sollbereichs den Wert -1 (hypoglykämischer Bereich) und oberhalb des Sollbereichs einen Wert von +1 hat (hyperglykämischer Bereich) hat.

Übersteigt die Anzahl der Zustandswechsel in einem betrachteten Zeitintervall einen vorgegebenen Schwellenwert, so deutet dies auf eine Instabilität in der Einstellung der Insulingaben hin und sollte deshalb in den Störungsparameter eingehen. Beispielsweise kann der Störungsparameter zusätzlich einen Wert enthalten, der die Anzahl der Zustandswechsel angibt oder aus dieser Anzahl berechnet wird.

Das beschriebene Verfahren kann von einem Analysehandgerät eingesetzt werden, mit dem Diabetiker selbständig ihren Blutzuckerspiegel überwachen. Auf diese Weise können einem Diabetiker durch das Handgerät wertvolle Hinweise gegeben werden, wie die erforderlichen Insulingaben besser an bestehende Bedürfnisse angepaßt werden können. Insbesondere kann ein Diabetiker durch Verwendung eines Handgeräts, welches das beschriebene Verfahren nutzt, selbständig kleinere Anpassungen der Einstellung vornehmen und - sofern größere Einstellungen erforderlich sind - an einen Arzt verwiesen werden.

In Fig. 6 sind die wesentlichen Komponeten eines solchen Geräts dargestellt. Eine Meßeinheit 1 mißt mit einem Sensor zu aufeinanderfolgenden Zeitpunkten tₙ Meßwerte. Dieses Meßsignal wird - im dargestellten Fall drahtlos - an einen Empfänger 2 übertragen von dem das Meßsignal an eine Auswerteeinheit 3 weitergeleitet wird, die einen Mikroprozessor 4 und einen Datenspeicher 5 enthält. Daten, beispielsweise über verabreichte Insulingaben oder aufgenommene Broteinheiten, und Befehle können auch über eine Eingabeeinheit 6 an die Auswerteeinheit übermittelt werden. Die Ausgabe von Resultaten erfolgt mittels einer Ausgabeeinheit 7, die ein Display und andere übliche Ausgabemittel einschließen kann. Selbstverständlich erfolgt die Datenverarbeitung der Auswerteeinheit 3 digital und es sind entsprechende Wandler zur Umwandlung analoger Signale in digitale Signale vorgesehen. Besonders gut sind implantierbare Sensoren geeignet, mit denen in relativ kurzen Zeitabständen, beispielsweise 5 min, Werte der Blutglucosekonzentration ermittelt werden können.

Das beschriebene Verfahren kann auch für eine Vorrichtung verwendet werden, die zusätzlich zu der Meßeinheit 2, dem Speicher 5 und der Auswerteeinheit 3 eine Insulinpumpe umfaßt, die von der Auswerteeinheit 3 unter Berücksichtigung der Empfehlung der ermittelten Klasse, welcher der Störungsparameter zugeordnet wurde, gesteuert wird.

Anstelle einer Insulinpumpe können auch andere Geräte zur Insulingabe verwendet werden, beispielsweise sogenannte Insulinpens, bei denen es sich um Injektionsgeräte von der Größe eines Kugelschreibers handelt. Bevorzugt wird die verabreichte Insulindosis drahtlos von dem zur Insulinabgabe verwendeten Gerät, beispielsweise die Insulinpumpe oder einem Insulinpen, an die Auswerteeinheit 3 übertragen. Dies kann beispielsweise nach jeder Insulingabe oder nach voreingestellten Zeitintervallen geschehen. Ergänzend können weitere Peripheriegeräte verwendet werden, beispielsweise Geräte, in denen Informationen über die Broteinheiten verschiedener Speisen gespeichert sind, so daß entsprechende Schätzwerte für eine eingenommene Mahlzeit auf Abruf an die Auswerteeinheit 3 des beschriebenen Geräts übertragen werden können. Selbstverständlich können entsprechende Informationen auch in einem Speicher der Auswerteeinheit 3 gespeichert sein.

## Patentansprüche

1. Verfahren zur Bewertung einer Serie von Werten der Glucosekonzentration eines Diabetikers für eine Einstellung der Dosierung von Insulingaben, wobei als Eingangsgrößen Werte der Glucosekonzentration g(t1) bis g(tn) verwendet werden, die sich auf Zeitpunkte t1 bis tn, die über einen Zeitraum von mindestens vier Stunden, vorzugsweise mindestens sechs Stunden verteilt sind, beziehen,
mit den folgenden Schritten:
- auf der Grundlage der Konzentrationswerte wird ein Störungsparameter ermittelt, der Art und/oder Schwere einer trotz Insulingaben vorhandene Störung des untersuchten Glucosestoffwechsels charakterisiert,
- der Störungsparameter wird anhand von vorgegebenen Parameterbereichen einer von mindestens zwei vorgegebenen Klassen zugeordnet, wobei den Klassen als Ausgangsgrößen jeweils Empfehlungen zur Einstellung der Dosierung der Insulingaben zugeordnet sind, wobei
- zum Ermitteln des Störungsparameters zunächst aus den Konzentrationswerten g(t₁) bis g(tₙ) Störungswerte w(g(t1)) bis w(g(tn)) als Funktionswerte einer Wertungsfunktion w berechnet werden
**dadurch gekennzeichnet, daß**
in einem weiteren Schritt aus den Störungswerten durch eine statistische Auswertung der Störungsparameter ermittelt wird, wobei die Wertungsfunktion in einem Sollbereich der Glucosekonzentration eine geringere Steigung als in einem an den Sollbereich angrenzenden Bereich hat

2. Verfahren nach Anspruch 1, **dadurch gekennzeichnet, daß** der Störungsparameter eine mehrdimensionale Größe mit mehreren Komponenten ist.

3. Verfahren nach einem der vorhergehenden Ansprüche, **dadurch gekennzeichnet, daß** die Berechnung des Störungsparameters einen Schritt umfaßt, in dem ein Mittelwert der Störungswerte berechnet wird.

4. Verfahren nach Anspruch 3, **dadurch gekennzeichnet, daß** die Berechnung des Störungsparameters einen Schritt umfaßt, in dem eine Standardabweichung der Störungswerte von dem Mittelwert berechnet wird.

5. Verfahren nach Anspruch 4, **dadurch gekennzeichnet, daß** eine Komponente des Störungsparameters ein Maß für die Standardabweichung der Störungswerte von dem Mittelwert ist.

6. Verfahren nach einem der Ansprüche 3 bis 5, **dadurch gekennzeichnet, daß** eine Komponente des Störungsparameters ein Maß für den Mittelwert der Störungswerte ist.

7. Verfahren nach einem der vorhergehenden Ansprüche, **dadurch gekennzeichnet, daß** die Berechnung des Störungsparameters einen Schritt umfaßt, in dem die Störungswerte jeweils einem von mehreren Zuständen zugeordnet werden und ermittelt wird, wie lange die verschiedenen Zustände jeweils andauern und/oder mit welcher Häufigkeit Zustandswechsel auftreten.

8. Verfahren nach Anspruch 7, **dadurch gekennzeichnet, daß** eine Komponente des Störungsparameters ein Maß für die Zeitspanne ist, über die einer der Zustände andauert.

9. Verfahren nach Anspruch 7 oder 8, **dadurch gekennzeichnet, daß** eine Komponente des Störungsparameters ein Maß für die Häufigkeit der Zustandswechsel ist.

10. Verfahren nach einem der vorhergehenden Ansprüche, **dadurch gekennzeichnet, daß** für mehrere Zeitintervalle, die jeweils in dem Zeitraum enthalten sind, jeweils aus den Störungswerten des betreffenden Intervalls eine Störungskennzahl berechnet wird und in einem weiteren Schritt aus den Störungskennzahlen der Störungsparameter ermittelt wird.

11. Verfahren nach Anspruch 10, **dadurch gekennzeichnet, daß** die Zeitintervalle, für welche die Störungskennzahlen jeweils berechnet werden, überlappen.

12. Verfahren nach einem der vorhergehenden Ansprüche, **dadurch gekennzeichnet, daß** die Wertungsfunktion, mit der die Störungswerte berechnet werden, eine abschnittsweise lineare Funktion ist.

13. Verfahren nach Anspruch 12, **dadurch gekennzeichnet, daß** die Wertungsfunktion in dem Sollbereich konstant ist.

14. Verfahren nach einem der vorhergehenden Ansprüche, **dadurch gekennzeichnet, daß** der Sollbereich zwischen 50 mg/dl und 250 mg/dl, vorzugsweise zwischen 70 mg/dl und 180 mg/dl, liegt.

15. Vorrichtung zur Bewertung einer Serie von Werten der Glucosekonzentration einer Körperflüssigkeit eines Diabetikers für eine Einstellung der Dosierung von Insulingaben, umfassend
- eine Messeinheit (1) zum Ermitteln von Werten der Glucosekonzentration g(t1) bis g(tn) für Zeitpunkte t1 bis tn, die über einen Zeitraum von mindestens vier Stunden, vorzugsweise von mindestens sechs Stunden, verteilt sind,
- einen Speicher (5) zum Speichern der Konzentrationswerte, und
- eine Auswerteeinheit (3) zum Auswerten der Konzentrationswerte, **dadurch gekennzeichnet, daß**
die Auswerteeinheit (3) so eingerichtet ist, daß sie im Betrieb durch Anwendung eines Verfahrens nach einem der vorhergehenden Ansprüche einen Störungsparameter ermittelt und einer von mindestens zwei vorgegebenen Klassen zuordnet, wobei den Klassen jeweils Empfehlungen hinsichtlich der optimalen Dosierung der Insulingaben zugeordnet sind, so daß diese Empfehlungen zur Einstellung der Dosierung der Insulingaben bereit gestellt werden können.

16. Vorrichtung nach Anspruch 15, **dadurch gekennzeichnet, daß** die Vorrichtung eine Eingabeeinheit (6) aufweist, über die für den zeitlichen Verlauf der Glucosekonzentration relevante Daten, insbesondere verabreichte Insulingaben und aufgenommene Broteinheiten, eingegeben oder von einem anderen Gerät empfangen werden können und die Auswerteeinheit (3) unter Verwendung dieser Daten und der den Klassen zugeordneten Empfehlungen benötigte Insulindosen berechnet.

17. Vorrichtung nach Anspruch 15 oder 16, **dadurch gekennzeichnet, daß** sie eine Ausgabeeinheit (7) zum Ausgeben einer Empfehlung hinsichtlich der Dosierung der Insulingabe des Diabetikers umfaßt.

18. Vorrichtung nach einem der Ansprüche 15 bis 17, **dadurch gekennzeichnet, daß** sie eine Insulinpumpe umfaßt, die von der Auswerteeinheit (3) unter Berücksichtigung der Empfehlung der Klasse, welcher der Störungsparameter zugeordnet wurde, gesteuert wird.

## Claims

1. Method for the assessment of a series of glucose concentration values of a diabetic for adjustment of the dosing of insulin administrations, whereby glucose concentration values g(t1) to g(tn) that are related to time points t1 to tn that are distributed over a period of time of at least four hours, preferably at least six hours, are used as input parameters,
comprising the following steps:
- based on the concentration values a disturbance parameter is determined that characterizes the type and/or severity of a disturbance of the tested glucose metabolism that is manifest despite the administration of insulin;
- the disturbance parameter is assigned by means of predetermined parameter ranges to one of at least two predetermined classes, to each of said classes recommendations for adjustment of the dosing of the insulin administrations being dedicated as output parameters, wherein
- in order to determine the disturbance parameter, first the concentration values g(t1) to g(tn) are used to calculate disturbance values w(g(t1)) to w(g(tn)) as function values of a weighting function w,
**characterized in that**
in a further step, a statistical analysis is used to determine the disturbance parameter from the disturbance values, wherein the weighting function has a lesser slope in a target range of the glucose concentration than in a range that is adjacent to said target range.

2. Method according to claim 1, **characterized in that** the disturbance parameter is a multi-dimensional parameter with multiple components.

3. Method according to any one of the preceding claims, **characterized in that** the calculation of the disturbance parameter comprises a step, in which a mean value of the disturbance values is calculated.

4. Method according to claim 3, **characterized in that** the calculation of the disturbance parameter comprises a step, in which a standard deviation of the disturbance values from said mean value is calculated.

5. Method according to claim 4, **characterized in that** a component of the disturbance parameter is a measure of the standard deviation of the disturbance values from the mean value.

6. Method according to any one of the claims 3 to 5, **characterized in that** a component of the disturbance parameter is a measure of the mean value of the disturbance values.

7. Method according to any one of the preceding claims, **characterized in that** the calculation of the disturbance parameter comprises a step, in which the disturbance values are assigned to one each of multiple states and it is determined how long the various states persist and/or how frequently changes of states occur.

8. Method according to claim 7, **characterized in that** a component of the disturbance parameter is a measure of the period of time for which one of these states persists.

9. Method according to claim 7 or 8, **characterized in that** a component of the disturbance parameter is a measure of the frequency of the changes of states.

10. Method according to any one of the preceding claims, **characterized in that**, for multiple time intervals that are each included in said period of time, the disturbance values of the respective time intervals are used to calculate a characteristic disturbance number and, in a further step, the characteristic disturbance numbers are used to determine the disturbance parameter.

11. Method according to claim 10, **characterized in that** the time intervals, for which the characteristic disturbance numbers each are calculated, overlap.

12. Method according to any one of the preceding claims, **characterized in that** the weighting function used to calculate the disturbance values is a function which is linear in sections.

13. Method according to claim 12, **characterized in that** the weighting function is constant in the target range.

14. Method according to any one of the preceding claims, **characterized in that** the target range is between 50 mg/dl and 250 mg/dl, preferably between 70 mg/dl and 180 mg/dl.

15. Device for assessment of a series of glucose concentration values of a body fluid of a diabetic for adjustment of the dosing of insulin administrations comprising
- a measuring unit (1) for determining glucose concentration values g(t1) to g(tn) for time points t1 to tn that are distributed over a period of time of at least four hours, preferably at least six hours;
- a memory (5) for storing the concentration values; and
- an analytical unit (3) for analyzing the concentration values,
**characterized in that**
the analytical unit (3) is adapted such that it can be operated to apply a method according to any one of the preceding claims to determine a disturbance parameter and assign it to one of at least two predetermined classes, to each of said classes recommendations concerning the optimal dosing of insulin administrations being dedicated such that these recommendations can be provided for adjustment of the dosing of the insulin administrations.

16. Device according to claim 15, **characterized in that** the device comprises an input unit (6) by means of which relevant data in terms of the time course of the glucose concentration, in particular with regard to insulin administrations given and bread units taken in, can be entered or received by another device, and **in that** the analytical unit (3) uses this data and the recommendations assigned to the classes to calculate required insulin doses.

17. Device according to claim 15 or 16, **characterized in that** it comprises an output unit (7) for output of a recommendation regarding the dosing of the insulin administration of the diabetic.

18. Device according to any one of claims 15 or 17, **characterized in that** it comprises an insulin pump that is controlled by the analytical unit (3) taking into consideration the recommendation of the class to which the disturbance parameter was assigned.

## Revendications

1. Procédé pour l'évaluation d'une série de valeurs de la concentration en glucose d'un diabétique pour le réglage du dosage de prises d'insuline, où on utilise comme grandeurs de départ des valeurs de la concentration en glucose g(t1) à g(tn) qui se rapportent à des moments t1 à tn, qui sont répartis sur une durée d'au moins quatre heures, de préférence d'au moins six heures, présentant les étapes suivantes :
- sur base des valeurs de concentration, on détermine un paramètre de trouble, dont le type et/ou la gravité caractérise un trouble existant du métabolisme du glucose analysé, malgré des prises d'insuline,
- le paramètre de trouble est associé à l'aide de plages de paramètre prédéfinies à une d'au moins deux classes prédéfinies, les classes étant associées, en tant que grandeurs de départ, à chaque fois à des recommandations pour le réglage du dosage des prises d'insuline, où
- pour la détermination du paramètre de trouble, on calcule d'abord à partir des valeurs de concentration g(t1) à g(tn) les valeurs de trouble w(g(t1)) à w(g(tn)) comme valeurs de fonction d'une fonction de pondération w
**caractérisé en ce qu'**on détermine dans une autre étape le paramètre de trouble à partir des valeurs de trouble, par une évaluation statistique, où la fonction de pondération présente, dans une plage de consigne de la concentration en glucose, une pente plus faible que dans une plage adjacente à la plage de consigne.

2. Procédé selon la revendication 1, **caractérisé en ce que** le paramètre de trouble est une grandeur multidimensionnelle avec plusieurs composants.

3. Procédé selon l'une quelconque des revendications précédentes, **caractérisé en ce que** le calcul du paramètre de trouble comprend une étape dans laquelle une moyenne des valeurs de trouble est calculée.

4. Procédé selon la revendication 3, **caractérisé en ce que** le calcul du paramètre de trouble comprend une étape dans laquelle une déviation standard des valeurs de trouble de la moyenne est calculée.

5. Procédé selon la revendication 4, **caractérisé qu'**un composant du paramètre de trouble est une mesure pour la déviation standard des valeurs de trouble de la moyenne.

6. Procédé selon l'une quelconque des revendications 3 à 5, **caractérisé en ce qu'**un composant du paramètre de trouble est une mesure de la moyenne des valeurs de trouble.

7. Procédé selon l'une quelconque des revendications précédentes, **caractérisé en ce que** le calcul du paramètre de trouble comprend une étape dans laquelle les valeurs de trouble sont à chaque fois associées à l'un parmi plusieurs états et on détermine combien de temps les différents états durent à chaque fois et/ou à quelle fréquence les changements d'état surviennent.

8. Procédé selon la revendication 7, **caractérisé en ce qu'**un composant du paramètre de trouble est une mesure du laps de temps que dure un des états.

9. Procédé selon la revendication 7 ou 8, **caractérisé en ce qu'**un composant du paramètre de trouble est une mesure de la fréquence des changements d'état.

10. Procédé selon l'une quelconque des revendications précédentes, **caractérisé en ce qu'**on calcule pour plusieurs intervalles de temps, qui sont à chaque fois contenus dans la durée, à chaque fois à partir des valeurs de trouble de l'intervalle concerné, un indice de trouble et on détermine dans une autre étape, à partir des indices de trouble, le paramètre de trouble.

11. Procédé selon la revendication 10, **caractérisé en ce que** les intervalles de temps pour lesquels on calcule à chaque fois des indices de trouble se chevauchent.

12. Procédé selon l'une quelconque des revendications précédentes, **caractérisé en ce que** la fonction de pondération avec laquelle les valeurs de trouble sont calculées est une fonction linéaire par intervalles.

13. Procédé selon la revendication 12, **caractérisé en ce que** la fonction de pondération est constante dans la plage de consigne.

14. Procédé selon l'une quelconque des revendications précédentes, **caractérisé en ce que** la plage de consigne est située entre 50 mg/dl et 250 mg/dl, de préférence entre 70 mg/dl et 180 mg/dl.

15. Dispositif pour évaluer une série de valeurs de concentration en glucose d'un liquide corporel d'un diabétique pour le réglage du dosage de prises d'insuline, comprenant
- une unité de mesure (1) pour la détermination de valeurs de la concentration en glucose g(t1) à g(tn) pour des moments t1 à tn, qui sont répartis sur une durée d'au moins quatre heures, de préférence d'au moins six heures,
- une mémoire (5) pour enregistrer les valeurs de concentration, et
- une unité d'évaluation (3) pour évaluer les valeurs de concentration,
**caractérisé en ce que** l'unité d'évaluation (3) est configurée de manière telle qu'elle détermine en fonctionnement, par l'utilisation d'un procédé selon l'une quelconque des revendications précédentes un paramètre de trouble et l'associe à une parmi au moins deux classes prédéfinies, les classes étant associées à chaque fois à des recommandations en ce qui concerne le dosage optimal des prises d'insuline, de manière telle que ces recommandations peuvent être mises à disposition pour le réglage du dosage des prises d'insuline.

16. Dispositif selon la revendication 15, **caractérisé en ce que** le dispositif présente une unité de saisie (6), via laquelle les données pertinentes pour l'évolution dans le temps de la concentration en glucose, en particulier les prises d'insuline administrées et les équivalents en hydrates de carbone absorbés, peuvent être introduites ou reçues d'un autre appareil et l'unité d'évaluation (3) calcule, en utilisant ces données et les recommandations associées aux classes, des doses d'insuline nécessaires.

17. Dispositif selon la revendication 15 ou 16, **caractérisé en ce qu'**il présente une unité de sortie (7) pour émettre une recommandation relative au dosage de la prise d'insuline d'un diabétique.

18. Dispositif selon l'une quelconque des revendications 15 à 17, **caractérisé en ce qu'**il comprend une pompe à insuline qui est contrôlée par l'unité de sortie (3) en tenant compte de la recommandation de la classe, à laquelle a été associée le paramètre de trouble.
